# EUROPÄISCHE PATENTSCHRIFT

(11) **EP 2 397 121 B1**
(45) Veröffentlichungstag und Bekanntmachung des Hinweises auf die Patenterteilung: **21.08.2013**
(21) Anmeldenummer: 11004879.0
(22) Anmeldetag: 15.06.2011
(51) Int. Cl.: A61K 9/20, A61K 9/50, A61K 31/198, A61K 31/275, A61K 45/06, A61K 31/137, A61K 31/277

(54) **Pharmazeutisches Präparat, welches Entacapon, Levodopa und Carbidopa enthält**
Pharmaceutical preparation containing Entacapone, Levodopa and Carbidopa
Préparation pharmaceutique contenant de l'entacapone, de la lévodopa et de la carbidopa

(30) Priorität: 15.06.2010 DE 102010023828
(43) Veröffentlichungstag der Anmeldung: 21.12.2011
(73) Patentinhaber: IIP - Institut für industrielle Pharmazie Forschungs- und Entwicklungsgesellschaft mbH, 55442 Stromberg (DE)
(72) Erfinder: Ludwig, Gerhard, Dr., 55442 Stromberg (DE); Kretzler, Kai, Dr., 63814 Mainaschaff (DE)
(74) Vertreter: Eisenführ, Speiser & Partner

(56) Entgegenhaltungen:
- WO-A1-2009/098663
- WO-A1-2010/020970
- WO-A2-2006/131591
- WO-A2-2007/069274
- US-A1- 2009 155 369

## Beschreibung

Die Erfindung betrifft ein pharmazeutisches Präparat und insbesondere ein festes orales Präparat, welches Entacapon, Levodopa und Carbidopa oder pharmazeutisch akzeptable Salze oder Hydrate davon enthält, sowie ein Verfahren zur Herstellung dieses Präparats.

Aus EP 1 189 608 B1 sind pharmazeutische Zusammensetzungen zur Behandlung von Parkinsonscher Krankheit bekannt. Bei einer Ausführungsform des bekannten oralen festen Präparats ist ein wesentlicher Anteil an Carbidopa von Entacapon und Levodopa getrennt. Ein weiteres orales festes Präparat enthält pharmakologisch wirksame Mengen an Entacapon, Levodopa und Carbidopa sowie einen Arzneimittelträger, der von microcrystalliner Cellulose verschieden ist.

WO 2010/020970 A1 beschreibt eine orale fest Arzneiform, welche in einer einzelnen Einheit, welche beispielsweise eine Mikrotablette sein kann, Levodopa, Carbidopa in einer verzögernd freisetzenden Form und Entacapon In einer sofort freisetzenden Form beinhaltet. Die einzelne Einheit kann als Zweischichttablette ausgebildet sein, wobei eine Schicht Entacapon und Hilfsstoffe wie Mannitol und alkalisierende Komponenten wie Carboxymethylstärke-Natrium und Croscarmellose-Natrium enthält. Die zweite Schicht beinhaltet Levodopa, Carbidopa und Hilfsstoffe.

WO 2007/069274 A2 beschreibt Zubereitungen, in denen Entacapon mit einer alkalisierenden Komponente, beispielsweise Carbonate, verarbeitet wird. Die Verarbeitung erfolgt mittels Nassgranulation. Entacapon kann mit Levodopa und Carbidopa separat oder in einer fixen Kombination verabreicht werden.

Aus WO 2009/098663 A1 sind Zubereitungen bekannt, in denen Entacapon mit einem Zuckeralkolhol durch Mikronisierung verarbeitet wird, zur Verbesserung der Lösungseigenschaften.

Aus US 2009/01 55369 A1 ist eine orale Darreichungsform bekannt, beispielsweise in Form einer Tablette, welche Entacapon, Levodopa und Carbidopa enthält. Entacapon liegt als Granulat vor und ist getrennt von den Wirkstoffen Levodopa und Carbidopa in der Tablette untergebracht Tablettierhilfsstoffe, wie beispielsweise Isomalt, bilden Komponenten des Arzneimittelträgers der gesamten Tablette. Hierbei soll ein verbessertes Löslichkeits- und Stabilitätsprofil erreicht werden.

WO 2006/131591 A2 beschreibt ein Kit aus einer Entacapon-Tablette und einer weiteren Dosisform mit Levodopa und Carbidopa. Die beschriebenen und gezeigten Tabletten haben relativ große Abmessungen.

Entacapon wird im US-Patent 5,446,194 als ein Catechol-O-methyltransferase-(COMT)-Inhibitor, beschrieben. Die chemische Bezeichnung von Entacapon ist (E)-2-Cyano-3-(3, 4-dihydroxy-5-nitrophenyl)-N,N-diethyl-2-propenamid und besitzt folgende Strukturformel:

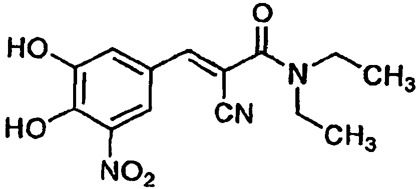

Carbidopa hat die chemische Bezeichnung (-)-L-α-Hydrazino-α-methyl-β-(3,4-dihydroxybenzol)propansäure monohydrat mit folgender Strukturformel:

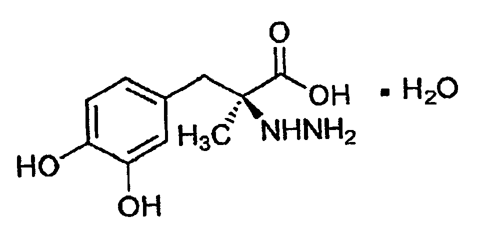

Levodopa hat die chemische Bezeichnung (-)-L-α-Amino-β-(3,4-dihydroxybenzol)propansäure mit folgender Strukturformel:

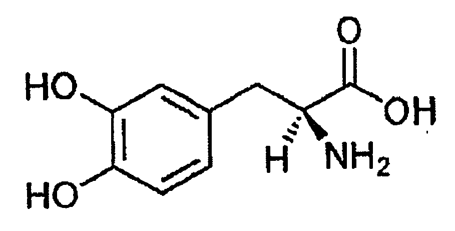

Aufgabe der Erfindung ist es ein pharmazeutisches Präparat der eingangs genannten Art zu schaffen, welches gut schluckbar ist und eine verbesserte Bioverfügbarkeit der aktiven Wirkstoffe, insbesondere von Entacapon, gewährleistet.

Diese Aufgabe wird durch ein orales festes Präparat gemäß Patentanspruch 1 gelöst, wobei in den Unteransprüchen vorteilhafte Weiterbildungen der Erfindung angegeben sind.

Bei der Erfindung sind in einer ersten Mikrotablette Levodopa und Carbidopa und in einer zweiten Mikrotablette Entacapon jeweils in zur therapeutischen Behandlung von Parkinson-Krankheit geeigneten Dosen mit pharmazeutisch akzeptablen Arzneimittelträgern enthalten.

Das Verhältnis der Dosen von Levodopa zu Carbidopa beträgt vorzugsweise etwa 4:1. Das Verhältnis der Dosen von Entacapon zu Levodopa kann ebenfalls etwa 4:1 betragen.

Vorzugsweise werden die beiden Mikrotabletten in einer gemeinsamen Hülle, beispielsweise in einem Beutel (Stick-Pack) oder Sachet für die jeweilige Verabreichung an den Parkinson-Patienten angeordnet. In bevorzugter Weise, weist jede der beiden Mikrotabletten einen wasserlöslichen Filmüberzug auf. Dieser kann beispielsweise aus Opadry ® II bestehen.

Zur Steuerung der in-vitro-Freisetzung kann die Levodopa und Carbidopa enthaltende Mikrotablette als Träger ein geeignetes viskoelastisches Polymer wie z.B. Polyvinylpyrrolidon, Hydroxypropylmethylcellulose, Hydroxyethylcellulose, Methylcellulose, Hydroxpropylcellulose, Ethylcellulose, Natriumcarboxymethylcellulose, Gelatine, Gummi arabicum enthalten, wobei mit Polymeren verschiedener Viskositätsklassen eine einer monolithischen Arzneiform gleicher Wirkstoffkonzentration entsprechende Freisetzung eingestellt werden kann. Der Arzneimittelträger kann außerdem wenigstens ein Antioxidanz, wie z.B. Citronensäure, Ascorbinsäure, Natriumdithitonit, Butylhydroxytoluol oder N-Acetylcystein aufweisen.

Ferner weist die Entacapon enthaltende Mikrotablette einen Arzneimittelträger auf, welcher während der in-vitro-Wirkstofffreisetzung den pH-Wert lokal ins Alkalische verschiebt. Der Wirkstoffträger weist hierzu Isomalt oder einen anderen Zucker oder Zuckeralkohol, wie Mannitol, Sorbitol, Lactitol, Xylitol, Erythritol, Saccarose, Lactose oder Maltose und wenigstens ein Carbonat von Alkimetallen oder Erdalkalimetalen, wie Natrium, Kalium, Calcium, Magnesium auf. Weitere geeignete Natriumverbindungen sind beispielsweise Natriumhydrogencarbonat, Tri-natriumphosphat, Natriumacetat, Tri-natriumcitrat. Durch die Verschiebung des pH-Wertes ins Alkalische erreicht man bei der in-vitro-Freisetzung eine deutlich verbesserte Löslichkeit von Entacapon. Hierdurch wird vermieden, dass die in-vitro-Freisetzung des Entacapons, welches kompaktiert ist, auch bei raschem Zerfallen der Mikrotablette nur sehr langsam vor sich geht.

Die Herstellung der Levodopa und Carbidopa enthaltenden Mikrotabletten erfolgt vorzugsweise mittels wässriger Granulation, während die Herstellung der Entacapon enthaltenden Mikrotabletten vorzugsweise mittels Trockenkompaktierung erfolgt.

Bei der wässrigen Granulation von Carbidopa und Levodopa werden Anteile des Arzneimittelträgers, beispielsweise ein pharmazeutisch akzeptables viskoelastisches Polymer, beispielsweise Hypromellose, und Citronensäure in der Granulierflüssigkeit gelöst. Nach der Trocknung und Siebung des Granulats können noch Kieselsäure und Magnesiumstearat nachgemischt werden. Anschließend wird die Mischung zu Mikrotabletten verpresst und noch mit einem wasserlöslichen Film, beispielsweise Film Opadry ® II überzogen.

Die Herstellung der Entacapon haltigen Mikrotablette erfolgt mit Trockenkompaktierung, wobei Anteile des Arzneimittelträgers und Entacapon gemischt und anschließend beispielsweise über einen Walzenkompaktor trocken kompaktiert werden. Das erhaltene Granulat kann im Freifall mit Kieselsäure und Magnesiumstearat nachgemischt und anschließend zu Mikrotabletten verpresst werden. Diese werden dann noch mit einem wasserlöslichen Filmüberzug, beispielsweise aus Film Opadry ® II überzogen.

Zur weiteren Erläuterung der Erfindung dienen die nachfolgenden Beispiele für die beiden Mikrotabletten:

### Levodopa / Carbidopa enthaltende Film-Minitabletten

### Beispiel 1, Rezeptur:

| **POS** | **Substanz** | **Menge (mg)** |
|---|---|---|
| 1 | Levodopa | 50 |
| 2 | Carbidopa monohydrat | 13,5 |
| 3 | Hypromellose Type 15 mPa*s | 20,18 |
| 4 | Citronensäure wasserfrei | 0,07 |
| 5 | Hypromellose Type 5 mPa*s | 1,95 |
| 6 | Wasser, gereinigt | 21,4 |
| 7 | Kieselsäure colloidal | 0,45 |
| 8 | Magnesium stearate | 2,2 |
| 9 | Film Opadry ® II | 8 |
| 10 | Wasser, gereinigt | 25 |

### Beispiel 2, Rezeptur:

| **POS** | **Substanz** | **Menge (mg)** |
|---|---|---|
| 1 | Levodopa | 50 |
| 2 | Carbidopa monohydrat | 13,5 |
| 3 | Hypromellose 4000 mPa*s | 20,18 |
| 4 | Citronensäure wasserfrei | 0,07 |
| 5 | Hypromellose 15 mPa*s | 1,7 |
| 6 | Wasser, gereinigt | 15 |
| 7 | Magnesium stearat | 1,06 |
| 8 | Opadry ® II | 8 |
| 9 | Wasser, gereinigt | 25 |

### Beispiel 3, Rezeptur:

| **POS** | **Substanz** | **Menge (mg)** |
|---|---|---|
| 1 | Levodopa | 50 |
| 2 | Carbidopa monohydrat | 13,5 |
| 3 | Hypromellose 4000 mPa*s | 10,08 |
| 4 | Hypromellose 50 mPa*s | 10,08 |
| 5 | Citronensäure wasserfrei | 0,07 |
| 6 | Hypromellose 15 mPa*s | 1,7 |
| 7 | Wasser, gereinigt | 15 |
| 8 | Magnesium stearat | 1,06 |
| 9 | Opadry ® II | 8 |
| 10 | Wasser, gereinigt | 25 |

### Beispiel 4, Rezeptur:

| **POS** | **Substanz** | **Menge (mg)** |
|---|---|---|
| 1 | Levodopa | 50 |
| 2 | Carbidopa monohydrat | 13,5 |
| 3 | Hypromellose 50 mPa*s | 20,18 |
| 4 | Citronensäure wasserfrei | 0,07 |
| 5 | Hypromellose 15 mPa*s | 1,7 |
| 6 | Wasser, gereinigt | 15 |
| 7 | Magnesium stearat | 1,06 |
| 8 | Opadry ® II | 8 |
| 9 | Wasser, gereinigt | 25 |

### Beispiel 5, Rezeptur:

| **POS** | **Substanz** | **Menge (mg)** |
|---|---|---|
| 1 | Levodopa | 50 |
| 2 | Carbidopa monohydrat | 13,5 |
| 3 | Hypromellose 15 mPa*s | 20,18 |
| 4 | Citronensäure wasserfrei | 0,07 |
| 5 | Hypromellose 15 mPa*s | 1,7 |
| 6 | Wasser, gereinigt | 15 |
| 7 | Magnesium stearat | 1,06 |
| 8 | Opadry ® II | 8 |
| 9 | Wasser, gereinigt | 25 |

Die Herstellung der Levodopa/Carbidopa enthaltenden Filmtabletten der obigen Beispiele erfolgt als wässrige Granulation. Hierbei werden zunächst die beiden Wirkstoffe und Hypromellose Pos 3 bzw. Hypromellosen Pos 3 und 4 (Bsp. 3) in einem Zwangsmischer trocken homogenisiert. Anschließend wird mit einer Lösung aus Hypromellose Pos 5 bzw. Pos 6 (Bsp.3) und Citronensäure und Wasser feucht granuliert. Nach der Trocknung und Siebung des Granulats wird noch die Kieselsäure und das Magnesiumstearat (Bsp.1) bzw. nur Magnesiumstearat im Freifall nachgemischt. Diese Mischung wird anschließend zu Mikrotabletten verpresst und noch mit dem wasserlöslichen Film überzogen.

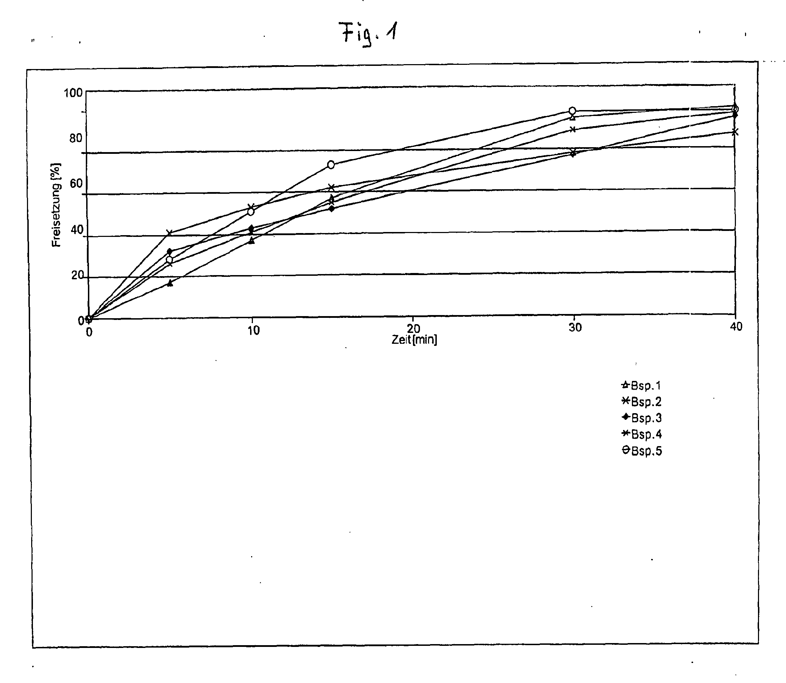

Freisetzungsprofile für die Beispiele 1 bis 5 der Levodopa-Carbidopa Filmtabletten sind in Figur 1 gezeigt. Hierbei wurden angewendet:

| | |
|---|---|
| Apparat | Drehkörbchen |
| Rührgeschwindigkeit | 100 Upm |
| Freisetzungs-Medium | 0,1 M Salzsäure |
| Freisetzungs-Volumen | 900 ml |
| Temperatur | 37°C |

Entacapon enthaltende Film-Minitabletten

### Beispiel 1, Rezeptur:

| **POS** | **Substanz** | **Menge (mg)** |
|---|---|---|
| 1 | Entacapon | 200 |
| 2 | Isomalt *r* | 115,5 |
| 3 | Natirumcarbonat | 59,5 |
| 4 | Povidone CL | 31 |
| 5 | Kieselsäure colloidal | 3 |
| 6 | Kieselsäure colloidal | 2 |
| 7 | Magnesium stearate | 4 |
| 8 | Film Opadry ® II | 31,8 |
| 9 | Wasser, gereinigt | 95,4 |

### Beispiel 2, Rezeptur:

| **POS** | **Substanz** | **Menge (mg)** |
|---|---|---|
| 1 | Entacapon | 200 |
| 2 | Isomalt | 115,5 |
| 3 | Natriumcarbonat | 40 |
| 4 | Povidone CL | 19,5 |
| 5 | Kieselsäure colloidal | 3 |
| 6 | Kieselsäure colloidal | 2 |
| 7 | Magnesium stearat | 4 |
| 8 | Opadry ® II | 31,8 |
| 9 | Wasser, gereinigt | 95,4 |

### Beispiel 3, Rezeptur:

| **POS** | **Substanz** | **Menge (mg)** |
|---|---|---|
| 1 | Entacapon | 200 |
| 2 | Isomalt | 115,5 |
| 3 | Natriumcarbonat | 59,5 |
| 4 | Povidone CL | 22 |
| 5 | Kieselsäure colloidal | 3 |
| 6 | Kieselsäure collodial | 2 |
| 7 | Magnesium stearat | 4 |
| 8 | Opadry ® II | 31,8 |
| 9 | Wasser, gereinigt | 95,4 |

### Beispiel 4, Rezeptur:

| **POS** | **Substanz** | **Menge (mg)** |
|---|---|---|
| 1 | Entacapon | 200 |
| 2 | Isomalt | 115,5 |
| 3 | Natriumcarbonat | 59,5 |
| 4 | Povidone CL | 43 |
| 5 | Kieselsäure colloidal | 3 |
| 6 | Kieselsäure colloidal | 2 |
| 7 | Magnesium stearat | 4 |
| 8 | Opadry ® II | 31,8 |
| 9 | Wasser, gereinigt | 95,4 |

Die Herstellung der Entacapon enthaltenden Film-Minitabletten obiger Beispiele 1 bis 4 erfolgt als Trockenkompaktierung. Dabei werden die ersten fünf Stoffe im Freifall gemischt und anschließend über einen Walzenkompaktor trocken kompaktiert. Das erhaltene Granulat wird mit Kieselsäure und Magnesiumstearat im Freifall nachgemischt und anschließend zu Mikrotabletten verpresst. Diese werden dann noch mit dem wasserlöslichen Film überzogen.

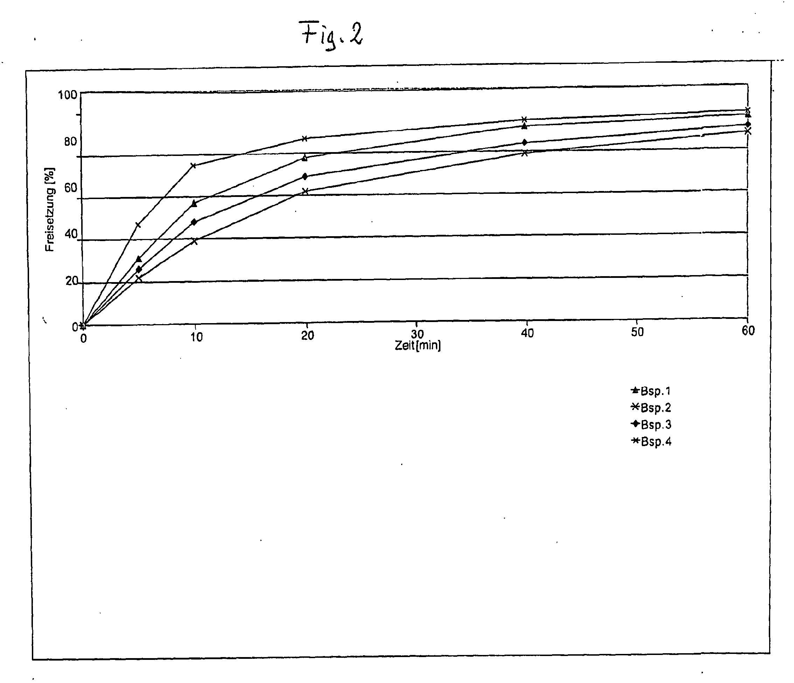

Freisetzungsprofile der Entacapon-Filmtabletten gemäß der Beispiele 1 bis 4 sind in Figur 2 gezeigt. Hierbei wurden angewendet:

| | |
|---|---|
| Apparat | Blattrührer |
| Rührgeschwindigkeit | 75 Upm |
| Freisetzungs-Medium | Puffer ph 5,8 |
| Freisetzungs-Volumen | 900 ml |
| Temperatur | 37°C |

Die Mikrotabletten haben größenordnungsmäßig einen Durchmesser von etwa 2mm und eine Höhe von etwa 2,5 mm. Abweichungen von diesen Abmessungen, welche die gewünschte Schluckfähigkeit nicht beeinträchtigen, sind möglich.

## Patentansprüche

1. Orales festes Präparat, welches pharmakologisch wirksame Mengen an Entacapon, Levodopa und Carbidopa sowie pharmazeutisch akzeptable Salze oder Hydrate davon und einen pharmazeutisch akzeptable Arzneimittelträger umfasst,
- Levodopa und Carbidopa in einer ersten Mikrotablette und Entacapon in einer zweiten Mikrotablette in zur therapeutischen Behandlung von Parkinson-Krankheit geeigneten Dosen vorliegen,
- die Entacapon enthaltende Mikrotablette kompaktiertes Entacapon und einen Arzneimittelträger aufweist, welcher während der Wirkstofffreisetzung den pH-Wert lokal ins Alkalische verschiebt, wobei
- die Entacapon enthaltende Mikrotablette in ihrem Arzneimittelträger wenigstens einen Zucker oder Zuckeralkohol, insbesondere Isomalt, und ein Carbonat von Alkalimetall oder Erdalkalimetall aufweist.

2. Präparat nach Anspruch 1, **dadurch gekennzeichnet, dass** die Dosen von Levodopa zu Carbidopa im Verhältnis von etwa 4:1 vorliegen.

3. Präparat nach Anspruch 1 oder 2, **dadurch gekennzeichnet, dass** die beiden Mikrotabletten in einer gemeinsamen Hülle, insbesondere Sachet vorgesehen sind.

4. Präparat nach einem der Ansprüche 1 bis 3, **dadurch gekennzeichnet, dass** die Mikrotabletten einen wasserlöslichen Filmüberzug aufweisen.

5. Präparat nach einem der Ansprüche 1 bis 4, **dadurch gekennzeichnet, dass** die Levodopa und Carbidopa enthaltende Mikrotablette einen Arzneimittelträger aufweist, der wenigstens eine die Wirkstofffreigabe steuernde Substanz, insbesondere ein viskoelasitsches Polymer enthält.

6. Präparat nach Anspruch 5, **dadurch gekennzeichnet, dass** das visko-elastische Polymer Hypromellose ist.

7. Präparat nach Anspruch 5 oder 6, **dadurch gekennzeichnet, dass** der Arzneimittelträger wenigstens ein Antioxidanz aufweist.

8. Präparat nach einem der Ansprüche 1 bis 7, **dadurch gekennzeichnet, dass** die Arzneimittelträger der beiden Mikrotabletten Kieselsäure und Magnesiumstearat aufweisen.

9. Verfahren zur Herstellung eines Präparats, gemäß einem der Ansprüche 1 bis 8, **gekennzeichnet durch**
a) Herstellung einer Levodopa und Carbidopa enthaltenden wässrigen Granulation, in deren Granulierflüssigkeit Anteile des Arzneimittelträges gelöst werden, und nach Trocknung und Siebung die Mischung des Granulats zu Mikrotabletten verpresst wird; und
b) Herstellung einer Mischung aus Entacapon und Anteile des Arzneimittelträgers mit anschließender Trockenkompaktierung und Verpressen des bei der Trockenkompaktierung erhaltenen Granulats zu Mikrotabletten.

10. Verfahren nach Anspruch 9, **dadurch gekennzeichnet, dass** bei beiden Herstellungsschritten vor dem Verpressen, Kieselsäure und Magnesiumstearat nachgemischt werden.

11. Verfahren nach Anspruch 9 oder 10, **dadurch gekennzeichnet, dass** nach dem Verpressen die jeweilige Tablette mit einem wasserlöslichen Film überzogen wird.

## Claims

1. A solid oral preparation, comprising pharmacologically effective amounts of entacapone, levodopa and carbidopa, pharmaceutically acceptable salts or hydrates thereof, and a pharmaceutically acceptable excipient,
- wherein the levodopa and carbidopa are provided in a first microtablet and the entacapone in a second microtablet, in dosages suitable for the therapeutic treatment of Parkinson's disease,
- wherein the microtablet containing entacapone includes compacted entacapone and an excipient which raises the pH locally to the alkali range during release of the active agent, wherein
- the microtablet containing entacapone includes at least one sugar or sugar alcohol, in particular Isomalt, and a carbonate of alkali metal or alkaline earth metal.

2. The preparation according to claim 1, **characterised in that** the dosages of levodopa and carbidopa are in a ratio of approximately 4:1.

3. The preparation according to claim 1 or 2, **characterised in that** the two microtablets are provided in a shared capsule, in particular a sachet.

4. The preparation according to any one of claims 1 to 3, **characterised in that** the microtablets have a water-soluble film coating.

5. The preparation according to any one of claims 1 to 4, **characterised in that** the microtablet containing levodopa and carbidopa includes an excipient which contains at least one substance, in particular a visco-elastic polymer, for controlling the release of active agent.

6. The preparation according to claim 5, **characterised in that** the visco-elastic polymer is hypromellose.

7. The preparation according to claim 5 or 6, **characterised in that** the excipient includes at least one anti-oxidant.

8. The preparation according to any one of claims 1 to 7, **characterised in that** the excipients of the two microtablets contain silicon dioxide and magnesium stearate.

9. A method for producing the preparation according to any one of claims 1 to 8, **characterised by**
a) producing an aqueous granulate containing levodopa and carbidopa, in the granulation liquid of which parts of the excipient are dissolved, and which is compressed to form microtablets after the granulate mixture has been dried and sieved; and
b) producing a mixture of entacapone and parts of the excipient, with subsequent dry compaction and compression of the granulate obtained by dry compaction to form microtablets.

10. The method according to claim 9, **characterised in that** silicon dioxide and magnesium stearate are added to the mixture before it is compressed.

11. The method according to claim 9 or 10, **characterised in that** the respective tablet is coated with a water-soluble film after compression.

## Revendications

1. Préparation solide pour la voie orale, qui comprend des quantités efficaces pharmacologiquement d'entacapone, de lévodopa et de carbidopa, ainsi que de leurs sels ou hydrates acceptables pharmaceutiquement, et un véhicule de médicament acceptable pharmaceutiquement,
- la lévodopa et la carbidopa se présentent dans un premier microcomprimé et l'entacapone dans un deuxième microcomprimé en des doses appropriées au traitement thérapeutique de la maladie de Parkinson,
- le microcomprimé contenant l'entacapone a de l'entacapone compactée et un véhicule de médicament, qui, pendant la libération de la substance active, met la valeur du pH localement dans le domaine alcalin, dans laquelle
- le microcomprimé contenant de l'entacapone a, dans son véhicule de médicament, au moins un sucre ou un alcool-sucre, notamment de l'isomalt, et un carbonate de métal alcalin ou de métal alcalinoterreux.

2. Préparation suivant la revendication 1, **caractérisée en ce que** les doses de la lévodopa par rapport à la carbidopa sont dans le rapport d'environ 4:1.

3. Préparation suivant la revendication 1 ou 2, **caractérisée en ce que** les deux microcomprimés sont prévus dans une enveloppe commune, notamment dans un sachet.

4. Préparation suivant l'une des revendications 1 à 3, **caractérisée en ce que** les microcomprimés ont un enrobage pelliculaire soluble dans l'eau.

5. Préparation suivant l'une des revendications 1 à 4, **caractérisée en ce que** les microcomprimés contenant la lévodopa et la carbidopa ont un véhicule de médicament, qui contient au moins une substance réglant la libération de principe actif, notamment un polymère viscoélastique.

6. Préparation suivant la revendication 5, **caractérisée en ce que** le polymère viscoélastique est l'hypromellose.

7. Préparation suivant la revendication 5 ou 6, **caractérisée en ce que** le véhicule de médicament comporte au moins un anti-oxydant.

8. Préparation suivant l'une des revendications 1 à 7, **caractérisée en ce que** le véhicule de médicament des deux microcomprimés comprend de l'acide silicique et du stéarate de magnésium.

9. Procédé de préparation d'une préparation suivant l'une des revendications 1 à 8, **caractérisé par**
a) la préparation d'une granulation aqueuse contenant de la lévodopa et de la carbidopa, dans le liquide de granulation de laquelle sont dissoutes des proportions du véhicule de médicament et, après séchage et criblage, le mélange du granulé est comprimé en microcomprimé ; et
b) la préparation d'un mélange d'entacapone et de proportions du véhicule de médicament avec ensuite compactage à sec et compression en microcomprimés du granulé obtenu lors du compactage à sec.

10. Procédé suivant la revendication 9, **caractérisé en ce que**, dans les deux stades de préparation, on mélange ensuite, avant la compression, de l'acide silicique et du stéarate de magnésium.

11. Procédé suivant la revendication 9 ou 10, **caractérisé en ce que**, après la compression, on enrobe les comprimés respectifs d'une pellicule soluble dans l'eau.
